Europäisches Patentamt

European Patent Office

Office européen des brevets

(19)

(11) Numéro de publication: 0 374 042
A2

# (12) DEMANDE DE BREVET EUROPEEN

(21) Numéro de dépôt: 89403461.0

(22) Date de dépôt: 13.12.89

(51) Int. Cl.⁵: **A61K 31/55**

(30) Priorité: 16.12.88 FR 8816606

(43) Date de publication de la demande:
20.06.90 Bulletin 90/25

(84) Etats contractants désignés:
**AT BE CH DE ES FR GB GR IT LI LU NL SE**

(71) Demandeur: **RHONE-POULENC SANTE**
**20, avenue Raymond Aron**
**F-92160 Antony(FR)**

(72) Inventeur: **Goni, Sylvie**
**93 Rue du bac**
**F-75007 Paris(FR)**
Inventeur: **Piot, Odile**
**91 Avenue d'Alfortville**
**F-94600 Choisy Le Roi(FR)**
Inventeur: **Zundel, Jean-Luc**
**85-87 Rue Saint Maur**
**F-75011 Paris(FR)**

(74) Mandataire: **Savina, Jacques et al**
**RHONE-POULENC SANTE, Service Brevets,**
**20 Avenue Raymond Aron**
**F-92165 Antony Cédex(FR)**

(54) **Application de la carpipramine pour obtenir un médicament destiné au traitement de l'anxiété et des troubles du sommeil.**

(57) Application de la carpipramine ou sel pharmaceutiquement acceptable de ce composé pour obtenir un médicament destiné au traitement de l'anxiété et des troubles du sommeil.

EP 0 374 042 A2

## APPLICATION DE LA CARPIPRAMINE POUR OBTENIR UN MEDICAMENT DESTINE AU TRAITEMENT DE L'ANXIETE ET DES TROUBLES DU SOMMEIL

La présente invention concerne l'application de la carpipramine ou un sel pharmaceutiquement acceptable de ce composé pour obtenir un médicament destiné au traitement de l'anxiété et des troubles du sommeil.

Du BSM 3872 M, il est connu que la carpipramine ou [(carbamoyl-4 pipéridino-4) pipéridino-3 propyl-1]-5 dihydro-10,11 dibenzo (b,f) azépine est utile dans le traitement des psychoses et notamment de la schizophrénie.

Il a maintenant été trouvé que la carpipramine ou un sel pharmaceutiquement acceptable de ce composé possède des propriétés antagonistes de la sérotonine (récepteurs 5HT2) et est utile dans le traitement de l'anxiété et des troubles du sommeil.

L'affinité de la carpipramine pour les sites récepteurs centraux à sérotonine (type $S_2$) a été déterminée selon une technique inspirée de celle de J.E. LEYSEN et al, Mol. Pharmacol. 21, 301 (1982) qui consiste à mesurer l'affinité du produit pour les sites de liaison de la kétansérine tritiée. Dans ce test, la $CI_{50}$ de la carpipramine est de 3 nM.

La carpipramine s'est également montrée antagoniste des secousses de la tête (head-twitches) à la mescaline chez la souris selon une technique inspirée de celle de S.J. CORNE et R.W. PICKERING, Psychopharmacologia, 11, 65-78 (1967). Dans ce test, la $DA_{50}$ de la carpipramine administrée par voie sous cutanée est de 8 mg/kg.

La carpipramine présente une toxicité faible. Sa $DL_{50}$ est égale à 250 mg/kg par voie orale chez la souris.

La carpipramine et ses sels pharmaceutiquement acceptables peuvent être préparés selon le procédé décrit dans le BSM 3872 M.

Comme exemples de sels pharmaceutiquement acceptables, on peut citer les sels d'addition avec les acides minéraux tels que chlorhydrate, sulfate, nitrate, phosphate, ou organiques tels que acétate, propionate, oxalate, succinate, benzoate, fumarate, maléate, méthanesulfonate, iséthionate, théophyllineacétate, salicylate, phénolphtalinate, méthylène-bis-$\beta$-oxynaphtoate ou des dérivés de substitution de ces composés.

Les médicaments destinés au traitement de l'anxiété et des troubles du sommeil sont constitués par la carpipramine ou un sel pharmaceutiquement acceptable de ce composé à l'état pur ou sous forme d'une composition dans laquelle il est associé à tout autre produit pharmaceutiquement compatible, pouvant être inerte ou physiologiquement actif. Ces médicaments peuvent être notamment utilisés par voie orale, parentérale ou rectale.

Comme compositions solides pour administration orale, peuvent être utilisés des comprimés, pilules, poudres (capsules de gélatine, cachets) ou granulés. Dans ces compositions, le principe actif est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

En thérapeutique humaine, la carpipramine ou un sel pharmaceutiquement acceptable de ce produit est utile dans le traitement de l'anxiété et des troubles du sommeil.

Les doses dépendent de l'effet recherché, de la durée du traitement et de la voie d'administration utilisée ; elles sont généralement comprises entre 10 et 400 mg par jour par voie orale pour un adulte avec des doses unitaires allant de 2 à 50 mg de substance active.

D'une façon générale, le médecin déterminera la posologie appropriée en fonction de l'âge, du poids et de tous les autres facteurs propres au sujet à traiter.

Les exemples suivants illustrent des compositions pharmaceutiques.

Exemple A

On prépare, selon la technique habituelle, des gélules dosées à 25 mg de produit actif ayant la composition suivante

| | |
|---|---|
| - carpipramine | 25 mg |
| - cellulose microcristalline | 75 mg |
| - mannitol | 41 mg |
| - silice colloïdale | 4 mg |
| - carboxyméthylamidon sodique | 25 mg |
| - talc | 18 mg |
| - stéarate de magnésium | 2 mg |
| - polyvidone excipient | 10 mg |

Exemple B

On prépare, selon la technique habituelle, des comprimés dosés à 50 mg de produit actif ayant la composition suivante:

| | |
|---|---|
| - carpipramine dichlorhydrate | 50 mg |
| - cellulose monocristalline | 75 mg |
| - mannitol | 41 mg |
| - polyvidone excipient | 10 mg |
| - carboxyméthylamidon | 25 mg |
| - silice colloïdale | 4 mg |
| - talc | 18 mg |
| - stéarate de magnésium | 2 mg |
| - mélange d'hydroxyméthylcellulose, glycérine, oxyde de titane (72-3,5-24,5) | q. s. p. 1 comprimé pelliculé terminé à 245 mg |

EXEMPLE C

On prépare une solution injectable contenant 10 mg de produit actif ayant la composition suivante :

| | |
|---|---|
| - carpipramine | 10 mg |
| - acide benzoïque | 80 mg |
| - alcool benzylique | 0,06 cm3 |
| - benzoate de sodium | 80 mg |
| - éthanol à 95 % | 0,4 cm3 |
| - hydroxyde de sodium | 24 mg |
| - propylène glycol | 1,6 cm3 |
| - eau | q. s. p. 4 cm3 |

## Revendications

1 - Application de la carpipramine ou un sel pharmaceutiquement acceptable de ce composé pour obtenir un médicament destiné au traitement de l'anxiété.

2 - Application de la carpipramine ou un sel pharmaceutiquement acceptable de ce composé pour obtenir un médicament destiné au traitement des troubles du sommeil.

4